# EUROPÄISCHE PATENTANMELDUNG

(11) **EP 3 760 242 A1**
(43) Veröffentlichungstag der Anmeldung: **06.01.2021**
(21) Anmeldenummer: 19183858.0
(22) Anmeldetag: 02.07.2019
(51) Int. Cl.: A61L 29/08

(54) **FUNKTIONALISIERTE BALLONOBERFLÄCHE**

(71) Anmelder: BIOTRONIK AG, 8180 Bülach (CH)
(72) Erfinder: Quint, Bodo, 79802 Dettighofen (DE); WERNLI, Jeremy, 5430 Wettingen (CH)
(74) Vertreter: Randoll, Sören

(57) **Zusammenfassung**

Die Erfindung betrifft ein Verfahren zur Herstellung eines Ballons (1) für einen Ballonkatheter (2), aufweisend die Schritte: Bereitstellen des Ballons (1), wobei der Ballon (1) eine äußere Oberfläche (1a) aufweist, Bereitstellen einer Lösung (13) aufweisend ein Lösungsmittel (11) und ein darin gelöstes Polymer (12), und Abscheiden des Polymers (12) auf die Oberfläche (1a) zum Ausbilden einer das Polymer (12) aufweisenden Oberflächenbeschichtung (10) auf der Oberfläche (1a) des Ballons (1).

## Beschreibung

Den Oberflächen von Ballons, die z.B. in Ballonkathetersystemen verwendet werden, z.B. zum Implantieren von Stents, kommt eine besondere Bedeutung zu, da durch eine entsprechende Modifizierung z. B. die Stenthaltekraft auf dem Ballon erhöht werden kann oder die Eigenschaften der Ballonoberfläche als Träger weiterer Komponenten, z.B. elektrische Leitungen oder Medikamentenbeschichtungen, verbessert werden können.

Bei bekannten Medikamenten-beschichteten Ballons besteht oftmals die Problematik, dass z.B. hierbei verwendete Coagulatbeschichtungen vergleichsweise dick ausfallen. Weiterhin besteht die Schwierigkeit, dass Trägermembranen oftmals zu fein strukturiert sind und daher nicht befähigt sind eine bestehende Beschichtungsrezeptur in ausreichender Weise aufzunehmen, so dass die strukturbedingte Haftvermittlung in einem solchen Fall nicht eintritt.

Bei Stent Delivery Systemen entstehen während der Stentexpansion intensive Scherkräfte, wobei oft die Haftung der Beschichtung zum Ballon selbst oder die Festigkeit der Beschichtung limitierend sind. Es besteht eine Gefährdung durch Abrasion der Beschichtung oder deren Delamination (Partikelemissionen).

Weiterhin verwenden Druckfarben und elektrisch druckbare Leiterbahnen regelmäßig milde und vorzugsweise unbedenkliche Lösungsmittelsysteme die für Polymersysteme eher weniger geeignet sind und selbst sehr leicht durch verbreitete Lösungsmittel wie Alkohole oder Aceton angelöst werden. Aus diesem Grunde werden diese technischen Systeme zum Teil zusätzlich durch einen chemischen Härtungsmechanismus verfeinert, der in der Regel thermisch ausgelöst wird. Da aber herkömmliche Leiterplattenmaterialien thermisch wesentlich stabiler sind als Ballonoberflächen sind die hier benötigten Prozessbedingungen nur schwierig umzusetzen. Bei ungenügender thermischer Fixierung werden diese gedruckten Strukturen durch folgende Auftragsschichten schnell angelöst.

Hiervon ausgehend liegt der vorliegenden Erfindung die Aufgabe zugrunde, ein Verfahren zur Herstellung eines Ballons für einen Ballonkatheter anzugeben, so dass eine Ballonoberfläche geschaffen wird, die eine erhöhte Reibung oder eine verbesserte Haftvermittlung zu Folgebeschichtungen oder einem gecrimpten Stent oder einen Schutz für eine weitere Komponente des Ballons bereitstellt.

Dieses Problem wird durch ein Verfahren mit den Merkmalen des Anspruchs 1, einen Ballon mit den Merkmalen des Anspruchs 13 sowie durch eine Verwendung mit den Merkmalen des Anspruchs 15 gelöst. Vorteilhafte Ausgestaltungen dieser Erfindungsaspekte sind in den entsprechenden Unteransprüchen angeben und werden nachfolgend beschrieben.

Demgemäß wird ein Verfahren zur Herstellung eines Ballons vorgeschlagen, insbesondere für einen Ballonkatheter, offenbart, aufweisend die Schritte:
- Bereitstellen des Ballons, wobei der Ballon eine äußere Oberfläche aufweist,
- Bereitstellen einer Lösung aufweisend ein Lösungsmittel und ein darin gelöstes Polymer, und
- Abscheiden des Polymers auf die Oberfläche zum Ausbilden einer das Polymer aufweisenden Oberflächenbeschichtung auf der Oberfläche des Ballons.

Das erfindungsgemäße Verfahren ermöglicht die Herstellung einer Oberflächenbeschichtung, die eine aufgeraute Oberfläche, welche z.B. aus einem polymeranalogen Material des Ballonmaterials besteht, aufweist. Dieses ist insbesondere inherent mit einem Basismaterial des Ballons über einen Quellungs- / Anlösungs-Vorgang verbunden. Die erzeugte Oberflächenbeschichtung weist in der Regel eine "vliesähnliche" Strukturierung auf, da selbst unter Sprühbedingungen durch die spritzende Wirkung und minimale Oberflächenspannung des verwendeten Lösungsmittels vorwiegend faserähnliche Partikelstrukturen erzeugt werden. Es können insbesondere Beschichtungswandstärken erreicht werden, die kleiner als 5 Mikrometer sind.

Gemäß einer Ausführungsform des Verfahrens ist das Lösungsmittel eine der folgenden Säuren: Trifluoressigsäure (C₂HF₃O₂, CAS-Nummer 76-05-1).

Weiterhin ist gemäß einer Ausführungsform des Verfahrens vorgesehen, dass das Polymer (11) eines der folgenden Polymere ist: ein aliphatisches oder aromatisches Polyamid, oder ein thermoplastisches Polymer wie ein Polyetherblockamid-Copolymer, wie PEBAX 6233.

Weiterhin ist gemäß einer Ausführungsform des Verfahrens vorgesehen, dass das Polymer in der Lösung eine Konzentration im Bereich von 1 Gew.-% bis 30 Gew-%, bevorzugt 1-10 Gew.-% aufweist. In einer Ausführungsform ist es bevorzugt, wenn das Polymer in der Lösung eine Konzentration im Bereich von 15 bis 25 Gew.-% aufweist. In einem solchen Bereich kann insbesondere ein Elektrospinningverfahren vorteilhaft ausgeführt werden. In einer anderen Ausführungsform ist es bevorzugt, wenn das Polymer in der Lösung eine Konzentration im Bereich von 1 bis 20 Gew.-% aufweist. In einer solchen Ausführungsform kann das Elektrosprühverfahren besonders vorteilhaft ausgeführt werden.

Weiterhin ist gemäß einer Ausführungsform des Verfahrens vorgesehen, dass die Lösung zum Abschieden des Polymers auf der Oberfläche des Ballons über eine Düse in Richtung auf die Oberfläche gesprüht wird, wobei insbesondere faserförmige Strukturen des Polymers gebildet werden. Die kann zum Beispiel durch ein Airbrush-Verfahren oder durch einen Elektrospinning-Prozess realisiert werden.

Weiterhin ist gemäß einer Ausführungsform des Verfahrens vorgesehen, dass die Düse einen Innendurchmesser im Bereich von 0,1 mm bis 1,2 mm aufweist.

Weiterhin ist gemäß einer Ausführungsform des Verfahrens vorgesehen, dass die Düse beim Sprühen der Lösung einen Abstand zur Oberfläche des Ballons aufweist der größer oder gleich 10 cm ist.

Gemäß einer weiteren bevorzugten Ausführungsform des Verfahrens ist vorgesehen, dass Polymer der Lösung faserförmig auf die Oberfläche des Ballons abgeschieden bzw. aufgebracht wird.

Weiterhin ist gemäß einer Ausführungsform des Verfahrens vorgesehen, dass der Ballon einen der folgenden Stoffe aufweiset oder aus einem der folgenden Stoffe gebildet ist: ein aliphatisches oder aromatisches Polyamid oder ein thermoplastisches elastomermodifiziertes Polymer wie zum Beispiel ein Polyetherblockamid (PEBA).

Weiterhin ist gemäß einer Ausführungsform des Verfahrens vorgesehen, dass die Oberflächenbeschichtung mit einem Medikament oder einer Zusammensetzung, die ein Medikament enthält, beschichtet wird.

Weiterhin ist gemäß einer Ausführungsform des Verfahrens vorgesehen, dass der Ballon auf der Oberfläche des Ballons vor dem Abscheiden des Polymers zumindest einen elektrischen Leiter aufweist, und wobei vorzugsweise die erzeugte Oberflächenbeschichtung normal zur Oberfläche des Ballons in einer Umgebung des mindestens einen Leiters über einen den mindestens einen Leiter bedeckenden Bereich der Oberflächenbeschichtung hinausragt. Der mindestens eine Leiter kann z.B. durch Bedrucken der Oberfläche des Ballons mit einer elektrisch leitfähigen Tinte erzeugt werden.

Ein weiterer Aspekt der vorliegenden Erfindung betrifft einen Ballon für einen Ballonkatheter, wobei der Ballon mittels des erfindungsgemäßen Verfahrens hergestellt ist.

Ein weiterer Aspekt der vorliegenden Erfindung betrifft einen Ballonkatheter, aufweisend einen erfindungsgemäßen Ballon sowie einen auf der Oberflächenbeschichtung des Ballons angeordneten Stent.

Ein weiterer Aspekt der vorliegenden Erfindung betrifft eine Verwendung einer Lösung aufweisend Trifluoressigsäure als Lösungsmittel sowie ein in dem Lösungsmittel gelöstes Polymer zum Beschichten einer äußeren Oberfläche eines Ballons.

Das Polymer kann in der Lösung die oben angegebene Konzentration aufweisen. Weiterhin kann es sich bei dem Polymer um eines der in diesem Zusammenhang oben angegebenen Polymere handeln.

Im Folgenden sollen Ausführungsformen sowie weitere Merkmale und Vorteile der Erfindung anhand der Figur erläutert werden. Es zeigt
- Fig. 1: eine schematische Darstellung einer Ausführungsform des erfindungsgemäßen Verfahrens;
- Fig. 2: eine fotographische Darstellung (Vergrößerung 200x) einer rauen, homogen verteilten Oberflächenbeschichtung aus einem Polyetherblockamid Pebax 6333, die mittels des erfindungsgemäßen Verfahrens erzeugt worden ist;
- Fig. 3: eine fotographische Darstellung (Vergrößerung 200x) einer mittels des erfindungsgemäßen Verfahrens erzeugten Umgebung einer gedruckten Leiterstruktur auf einem Ballon; und
- Fig. 4: eine fotographische Darstellung (Vergrößerung 50x) einer mittels des erfindungsgemäßen Verfahrens erzeugten Oberflächenbeschichtung.

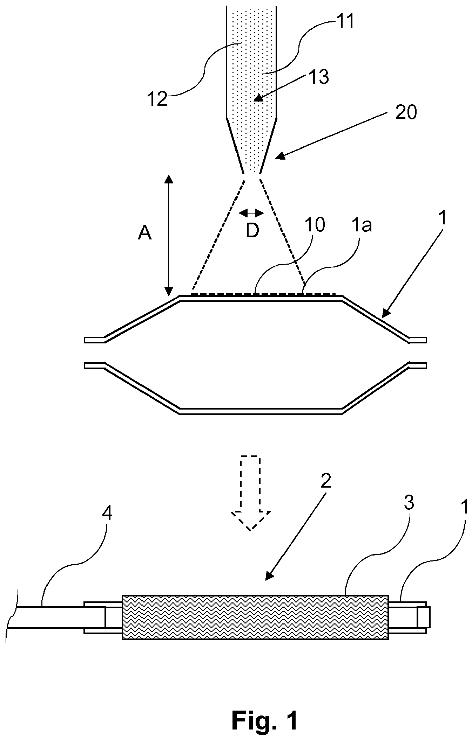

Figur 1 zeigt eine schematische Darstellung einer Ausführungsform eines erfindungsgemäßen Verfahrens zur Herstellung eines Ballons 1 für einen Ballonkatheter 2, aufweisend die Schritte:
- Bereitstellen des Ballons 1, wobei der Ballon 1 eine äußere Oberfläche 1a aufweist,
- Bereitstellen einer Lösung 13 aufweisend ein Lösungsmittel 11 und ein darin gelöstes Polymer 12, wobei hier z.B. als Polymer 12 Pebax 6233 in einer Konzentration von 1 Gew.-% bis 5 Gew.-% in Trifluoressigsäure gelöst wird, und
- Abscheiden des Polymers 12 auf die Oberfläche 1a zum Ausbilden einer das Polymer 12 aufweisenden Oberflächenbeschichtung 10 auf der Oberfläche 1a des Ballons 1.

Die Trifluoressigsäure besitzt in dieser Hinsicht mit Vorteil ein sehr ungewöhnliches Benetzungsverhalten, da diese Verbindung auf einem sehr kleinen Molekül basiert, das an einem Ende "ultrahydrophob" ist und auf dem entgegengesetzten Molekülende eine "hochpolare" organische Säure darstellt. Sie hat aufgrund dieser Struktur zum Beispiel die Angewohnheit, aus Glas-/Prozellangefäßen regelrecht herauszukriechen. Trotz der sauren Wirkung und der hohen Polarität bildet diese Verbindung Oberflächen mit sehr geringer Oberflächenspannung und sehr guter Benetzungswirkung aus.

Eine Vielzahl von Polyamiden, wie auch die im Katheterbau typischen PEBA-Copolymere, sind in Trifluoressigsäure löslich. Das Besprühen von Oberflächen 1a solcher Ballons mit der vorgenannten Lösung 13 führt zu überraschenden Ergebnissen.

So konnten z.B. bei einem Sprühabstand A der Düse 20 zur Oberfläche 1a größer gleich 10 cm und einem Innendurchmesser D der Düse 20 im Bereich von 0,1 mm bis 1,2 mm sowie einem Sprühdruck von ca. 3 bar gelförmige Abscheidungen auf der Oberfläche 1a beobachtet werden, die noch Relaxationen am Ballon 1 erzeugen, diesen aber kaum mehr intensiv verformen. Unter einem Stützdruck des Ballons aus PA12 von 1 bar wurden trotz einseitigen Besprühens gerade, nicht merklich deformierte Bauteile erhalten. Überraschend war jedoch das über einen sehr großen Verdünnungsbereich immer noch raue Oberflächenabscheidungen erhalten wurden, die eine vliesähnliche Struktur aufweisen.

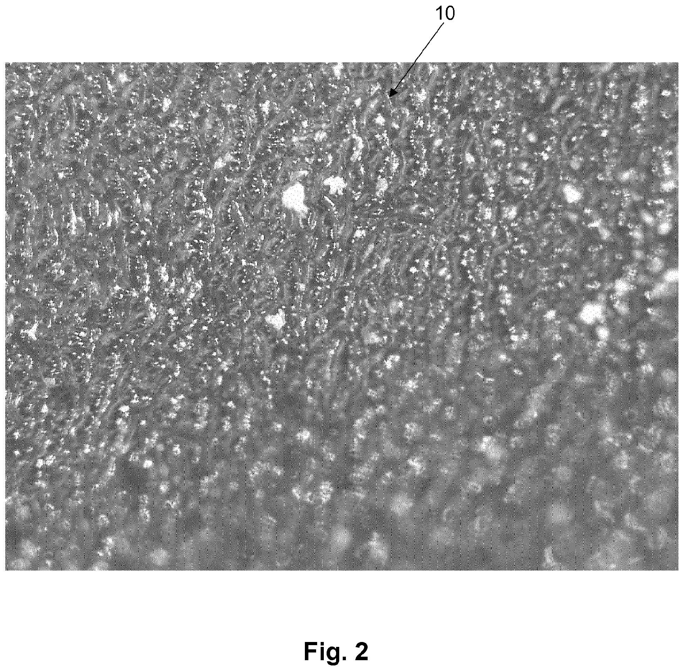

In den Figuren 2 bis 4 ist diese Struktur durch Einfärben der Beschichtungslösung 13 mit Rhodamin kenntlich gemacht. Bei Vergrößerungen >> 100-fach zeigt sich deutlich eine komplexe im Mikrometerbereich strukturierte Oberflächenstruktur (vgl. Figur 2).

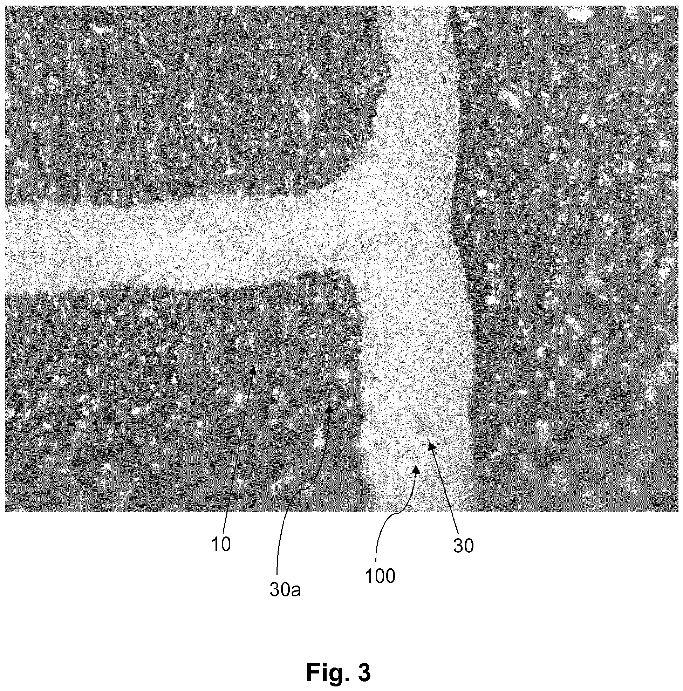

Der zu beschichtende Ballon 1 kann des Weiteren eine z.B. gedruckte Leiterstruktur, z.B. in Form zumindest eines elektrischen Leiters 30 aufweisen. Figur 3 zeigt diesbezüglich, dass die Oberflächenbeschichtung 10 auf dem Ballon 1 in der Umgebung der Leiter 30 durch Lösungsmittelverlust zu dem unterliegenden Polyamid spontan geliert, während der Benetzungseffekt auf dem jeweiligen Leiter 30 überwiegt, was zu einer flächig und dünnen isolierenden Beschichtung 100 auf diesem Untergrund führt. Mechanisch wird somit der jeweilige Leiter 30 mit einem hochfesten, flexiblen Material in die neu entstandene Oberfläche eingebettet und kann somit vor Peeling oder scherender Belastung geschützt werden.

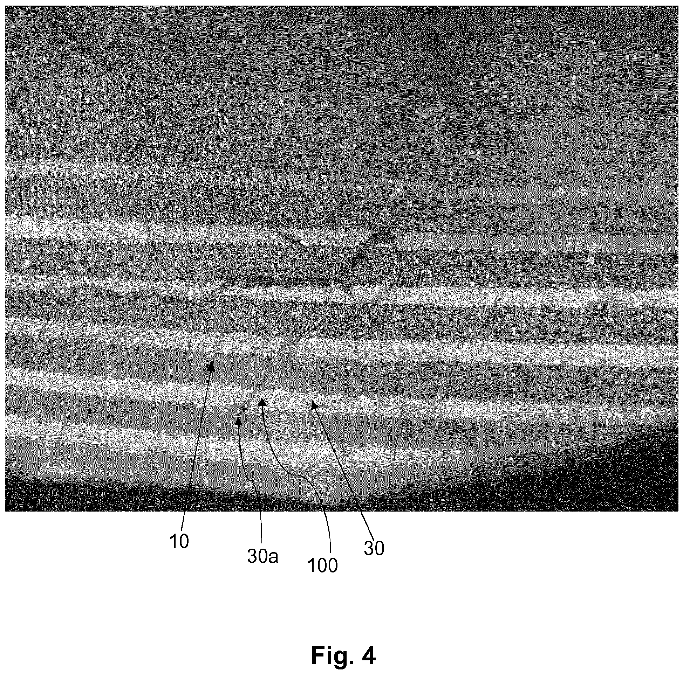

Gemäß Figur 4 (Vergrößerung 50x) weist bei der umgesetzten Sprühbeschichtung mit einer nadelförmigen Düsenanordnung auch der umgebende und weiter entfernte Sprühbereich spinnennetzartige Ablagerungen auf.

Der Sprühprozess (und die genutzte Düsengeometrie) erzeugen aufgrund der geringen Oberflächenspannung der Trifluoressigsäure und der Viskosität der Polymerlösungen vorzugsweise fadenförmige Fragmente, die in der Gasphase durch Lösungsmittelverlust erhalten bleiben.

Aufgrund der geringen Oberflächenspannung wird das Polymer 12 in der scharf auslaufenden Düse 20 fadenförmig abgezogen und diese Struktur wird aufgrund des hohen Dampfdruckes während des Aufenthaltes in der Sprühphase annähernd unverändert beibehalten. Diese faserförmigen Partikel werden in gelartiger Form an der Oberfläche 1a abgelegt und erleiden dort noch einen erheblichen Volumenverlust durch den Trocknungsvorgang der durch die saugende bzw. lösungsmittelabziehende Wirkung der Polyamid-Ballonoberfläche 1a verstärkt wird.

Da Trifluoressigsäure ein intensives Lösungsmittel für Polyamid 12 darstellt, erzeugt diese Restfeuchte eine Lösungsmittelverklebung zum Untergrund. Diese Oberfläche ist selbst durch intensive abrasive Einwirkung von der Oberfläche 1a nicht abreibbar / trennbar.

Diese Eigenschaften, die geringe Oberflächenspannung und sehr spontane Gelbildung, prädestinieren die Lösung 13 für einen Elektrospinn- oder Elektrosprühprozess. Da hier sowohl kleinere Materialtransfermengen erzielbar sind und eine höhere Kontrolle über die Abscheidung erreichbar ist, ist das Elektrospinnverfahren eine der bevorzugten Methoden zum Aufbringen der Oberflächenbeschichtung 10.

Wie weiterhin in Fig. 1 gezeigt ist, eignet sich die hergestellte Oberflächenstruktur insbesondere auch zur Erhöhung der erzielbaren Stenthaltekraft bzw. zur Haftungserhöhung für eine Medikamentenbeschichtung.

In weiteren Schritten des Verfahrens kann entsprechend der Ballon 1 zu einem Katheter 2 komplettiert werden. Der Katheter 2 erhält dabei einen Schaft 4, wobei der Ballon 1 am distalen Ende des Schafts 4 festgelegt wird, und wobei auf den gefalteten Ballon 1 bzw. auf die Oberflächenbeschichtung 10 z.B. ein Stent 3 gecrimpt werden kann. Anstelle eines Stents 3 kann die Oberflächenbeschichtung 10 des Ballons 1 eine Medikamentenbeschichtung erhalten.

Die erfindungsgemäß herstellbaren Oberflächenbeschichtungen 10 ermöglichen mit Vorteil ein Einbringen einer aufgebrachten Wirkstoffschicht in die neu geschaffene Oberflächenstruktur und erlauben daher ein Aufbringen großer Mengen eines Wirkstoffes in die Unebenheiten der Ballonbeschichtung.

Bei Ballonkathetern 2 kann der Reibungsgewinn zu der Stentoberfläche zu einer verbesserten Stenthaltekraft beitragen.

Die elektrische Isolationswirkung ist letztendlich durch die Oberflächenrauigkeit und Struktur der erzeugten Beschichtung limitiert, aber es ist dennoch erkennbar (vgl. Figuren 3 und 4), dass nach Abscheidung flächig geschlossene und dünne Beschichtungen erzielbar sind. Vorteilhaft ist weiterhin, dass das Lösungsmittelsystem 13 nicht mit den typischen acrylatbasierten Bindemitteln interagiert, wodurch ermöglicht wird, kommerziell verfügbare elektrisch leitende Tinten ohne intensive thermische Härtung und nachteilige Löseeffekte auf die jeweilige Ballonoberfläche aufzubringen. Eine Besonderheit besteht dabei in dem erreichbaren mechanischen Schutz dieser gedruckten Strukturen 30, da die umgebende Oberflächenbeschichtung 10 schneller an Dicke gewinnt als auf den gedruckten Strukturen 30 selbst.

Durch den rau strukturierten Aufbau 10 benachbart zu den Leiterbahnen 30 wird ein erhöhter Dickenaufbau im Vergleich zur jeweiligen Leiterbahn 30 erreicht. Das bedeutet, dass ursprünglich erhabene leitende elektrische Strukturen 30 nach erfolgter Beschichtung mechanisch geschützt und eingebettet vorliegen.

## Patentansprüche

1. Verfahren zur Herstellung eines Ballons (1) für einen Ballonkatheter (2), aufweisend die Schritte:
- Bereitstellen des Ballons (1), wobei der Ballon (1) eine äußere Oberfläche (1a) aufweist,
- Bereitstellen einer Lösung (13) aufweisend ein Lösungsmittel (11) und ein darin gelöstes Polymer (12), und
- Abscheiden des Polymers (12) auf die Oberfläche (1a) zum Ausbilden einer das Polymer (12) aufweisenden Oberflächenbeschichtung (10) auf der Oberfläche (1a) des Ballons (1).

2. Verfahren nach Anspruch 1, wobei das Lösungsmittel (11) eine der folgenden Säuren ist: Trifluoressigsäure.

3. Verfahren nach einem der vorhergehenden Ansprüche, wobei das Polymer (12) eines der folgenden Polymere ist: ein aliphatisches oder aromatisches Polyamid, oder ein thermoplastisches Polymer, vorzugsweise ein thermoplastisches elastomermodifiziertes Polymer, vorzugsweise ein Polyetherblockamid.

4. Verfahren nach einem der vorhergehenden Ansprüche, wobei das Polymer (12) in der Lösung (13) eine Konzentration im Bereich von 1 Gew.-% bis 30 Gew-% aufweist.

5. Verfahren nach einem der vorhergehenden Ansprüche, wobei dass Polymer (12) der Lösung (13) mittels Elektrospinnen auf die Oberfläche (1a) abgeschieden wird.

6. Verfahren nach einem der vorhergehenden Ansprüche, wobei der Ballon (1) einen der folgenden Stoffe aufweiset oder aus einem der folgenden Stoffe gebildet ist: ein aliphatisches oder aromatisches Polyamid, oder ein thermoplastisches elastomermodifiziertes Polymer wie z.B. ein Polyetherblockamid.

7. Verfahren nach einem der vorhergehenden Ansprüche, wobei die Oberflächenbeschichtung (10) mit einem Medikament oder einer Zusammensetzung, die ein Medikament enthält, beschichtet wird.

8. Verfahren nach einem der vorhergehenden Ansprüche, wobei der Ballon (1) auf der Oberfläche (1a) des Ballons vor dem Abscheiden des Polymers (12) zumindest einen elektrischen Leiter (30) aufweist, und wobei insbesondere die erzeugte Oberflächenbeschichtung (10) normal zur Oberfläche (1a) des Ballons (1) in einer Umgebung (30a) des mindestens einen Leiters (30) über einen den mindestens einen Leiter (30) bedeckenden Bereich (100) der Oberflächenbeschichtung (10) hinausragt.

9. Ballon (1) für einen Ballonkatheter (2), wobei der Ballon (1) mittels des Verfahrens nach einem der vorhergehenden Ansprüche hergestellt ist.

10. Ballonkatheter (2), aufweisend einen Ballon (1) nach Anspruch 9 sowie einen auf der Oberflächenbeschichtung (10) des Ballons (1) angeordneten Stent (3).

11. Verwendung einer Lösung (13) aufweisend Trifluoressigsäure als Lösungsmittel (11) sowie ein in dem Lösungsmittel (11) gelöstes Polymer (12) zum Beschichten einer äußeren Oberfläche (1a) eines Ballons (1).
